# EUROPEAN PATENT SPECIFICATION

(11) **EP 0 797 164 B1**
(45) Date of publication and mention of the grant of the patent: **29.03.2000**
(21) Application number: 97301142.2
(22) Date of filing: 21.02.1997
(51) Int. Cl.: G06F 17/60

(54) **Appointment booking and scheduling system**
Terminbuchung und -planungssystem
Système pour l'enregistrement et la planification de rendez-vous

(30) Priority: 23.03.1996 GB 9606194
(43) Date of publication of application: 24.09.1997
(73) Proprietor: INTERNATIONAL COMPUTERS LIMITED, Putney, London, SW15 1SW (GB)
(72) Inventor: Edgar, James William Hardie, Uphall Station, West Lothian EH54 5QJ (GB); Gathercole, Peter John, Cookridge, Leeds LS16 7DJ (GB); Johnson, Michael Alan, Gerrards Cross SL9 0DL (GB); Roycroft, Alistair Edward, Withington, Manchester M20 4GH (GB); Smith, Alan, Ladybridge, Bolton BL3 4NN (GB); Webster, Donald Brian, Bracknell, Berks RG42 2LE (GB)
(74) Representative: Guyatt, Derek Charles

(56) References cited:
- EP-A- 0 669 586
- EP-A- 0 672 990
- WO-A-95/26535
- IEEE EXPERT, vol. 9, no. 2, 1 April 1994, pages 33-39, XP000486333 COLLINS J E ET AL: "AUTOMATED ASSIGNMENT AND SCHEDULING OF SERVICE PERSONNEL"
- BT TECHNOLOGY JOURNAL, vol. 13, no. 1, 1 January 1995, pages 81-94, XP000486261 AZARMI N ET AL: "WORKFORCE SCHEDULING WITH CONSTRAINT LOGIC PROGRAMMING"
- BT TECHNOLOGY JOURNAL, vol. 13, no. 1, 1 January 1995, pages 46-54, XP000486257 LAITHWAITE R: "WORK ALLOCATION CHALLENGES AND SOLUTIONS IN A LARGE-SCALE WORK MANAGEMENT ENVIRONMENT"

## Description

### Background to the Invention

This invention relates to a computerised appointment booking and scheduling apparatus for scheduling a plurality of operatives (e.g. service technicians) travelling to sites (e.g. customer sites) within a geographical area.

Laithwaite, "Work allocation challenges and solutions in a large-scale work management environment", BT Technology Journal, Vol.13, No.1, January 1995, pages 46-54 describes a computerised work allocation system for field engineers. The system comprises a job reception component for receiving new jobs and adding them to a pool of work, and a tour-build algorithm for using the pool of jobs to generate a number of optimised tours for the engineers. The system also includes a real-time algorithm to cope with reactive work.

Collins and Sisley, "Automated assignment and scheduling of service personnel", IEEE Expert, Vol.2, No.9, April 1994, pages 33-39 describes a computerised service management system which maintains current schedules for each of a number of technicians. The system uses an incremental scheduling system to update the schedules when a new service call is received or when a call is removed.

The object of the invention is to provide an improved appointment booking and scheduling apparatus which is able to offer appointments within specific time slots.

### Summary of the Invention

According to the invention there is provided a computerised appointment booking and scheduling apparatus for scheduling a plurality of operatives travelling to sites in a geographical area, the apparatus comprising a database containing a pool of jobs, input means for adding new jobs to said pool, and scheduling means for using the pool of jobs to generate and to store in the database a plurality of routes, indicating sequences of sites to be visited by said operatives, characterised in that:
(a) said geographical area is divided into a plurality of regions, each of which regions includes a plurality of said sites;
(b) each of said routes comprises information specifying a sequence of said regions to be visited by a particular operative, the times the operative is scheduled to enter and leave each of said sequence of regions, and the amount of free time available for new appointments in each of said sequence of regions;
(c) said input means comprises means for offering appointments at specific times, using the currently stored routes to check for availability of operatives in specific regions at specific times, and for updating said pool of jobs and said routes to reflect new appointments that have been accepted; and (d) the apparatus also includes means for periodically running said scheduling means to generate from scratch a new plurality of routes, optimising the new plurality of routes to minimise a cost function, and storing them in the database to replace the currently stored routes.

The invention also considers a method of operating a computer apparatus as claimed in claim 5.

### Brief Description of the Drawings

Figure 1 is a block diagram of an appointment booking and scheduling system in accordance with the invention.

Figure 2 is a schematic representation of a geographic area within which appointments are to be booked.

Figure 3 is a schematic representation of a database holding a number of routes.

Figure 4 is a flow chart showing the operation of an appointment server.

Figure 5 is a flow chart showing the operation of an appointment booking scheduler.

Figure 6 is a flow chart showing an optimization process performed by the appointment booking scheduler.

### Description of an Embodiment of the Invention

One appointment booking and scheduling system in accordance with the invention will now be described by way of example with reference to the accompanying drawings. The system to be described is intended to handle appointments for a number of operatives (e.g. service engineers), visiting customer sites within a defined geographic area.

### Overview of the system

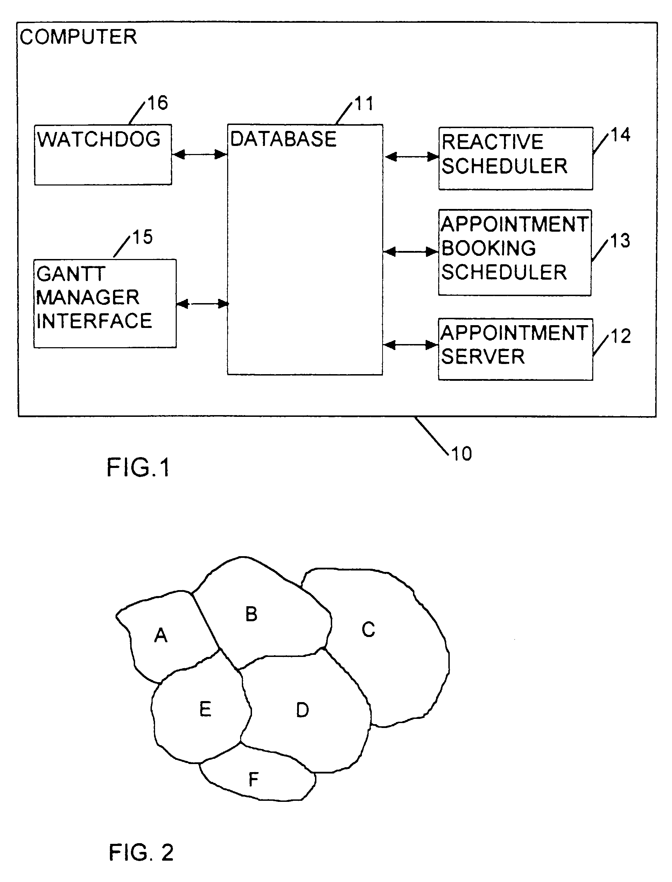

Referring to Figure 1, the appointment booking and scheduling system comprises conventional computer hardware 10, having a processor, memory, display, input device, and disks (not shown). The computer hardware stores a database 11, and runs the following software: an appointment server 12, an appointment booking scheduler 13, a reactive scheduler 14, a Gantt manager interface 15, and a watchdog 16.

Referring to Figure 2, the area in which appointments are to be booked is divided into a number of regions, designated A, B, C ... Each of these regions includes a number of customer sites, the location of each of which may, for example, be indicated by its postcode.

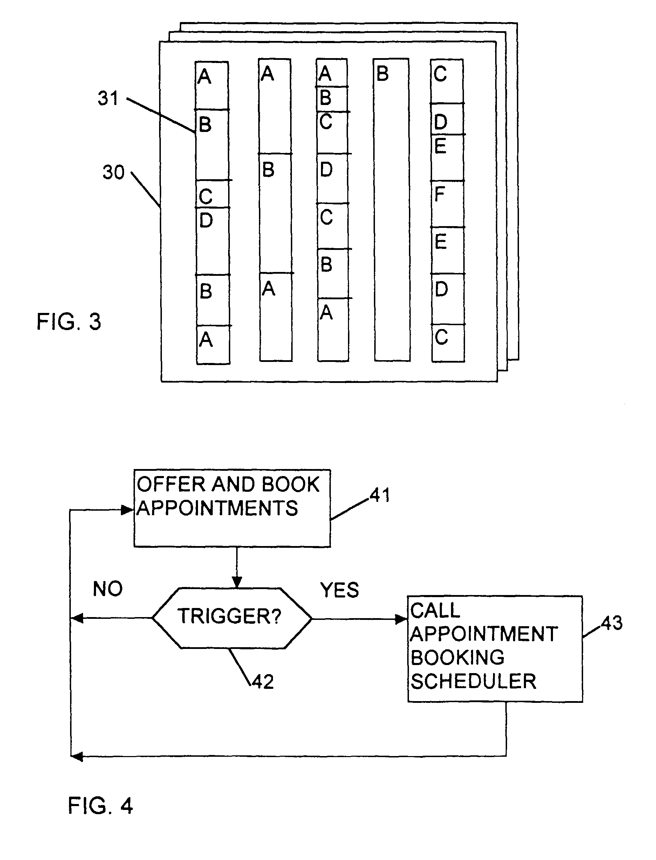

Referring to Figure 3, the database 11 includes a number of tables 30, one for each day in a predetermined window in which appointments may be offered. For example, this window may cover two weeks from the current date. Each of the tables 30 contains a number of routes 31. Each route represents an itinerary for a particular operative on the day in question, and has a number of jobs associated with it. Each route comprises a sequence of records, with each record containing the following fields:-
Region: the identity of a region to be visited by the operative.
Start Time: the time the operative is scheduled to start working in the region.
End Time: the time the operative is scheduled to finish working in the region.
Time Used: the number of minutes scheduled to be spent on jobs already booked within the region.
Time Left: the number of minutes free, available for new jobs in the region.

The sum of Time Used and Time Left will normally equal the difference between Start Time and End Time.

The database 11 also includes a number of job tables, which store a record for each job that has been booked. Each job record includes:
- details of the customer
- details of the problem
- promised time slot
- the route to which the job is currently allocated.

### Appointment server

Figure 4 shows the operation of the appointment server 12.

(Step 41) The appointment server uses the routes stored in the database to offer a number of possible appointments to customers. Specifically, if a customer within a particular region requests an appointment, the appointment server searches the routes to find one which visits the region and which contains sufficient free time within that region. Appointments are offered within predetermined time slots, e.g. two hour slots.

If the customer accepts the offered appointment, the Time Used and Time Left fields of the appropriate record within the selected route are updated. Details of the job, including the selected route, are entered in the job tables in the database.

(Step 42) When an appointment is made, the appointment server checks whether a predetermined trigger number of appointments has been made since the last time the scheduler was run. If not, the appointment server returns to Step 41 to await the next appointment.

(Step 43) When the trigger number of appointments has been made, the appointment server calls the scheduler, and immediately returns to Step 41 to await the next appointment.

### Appointment booking scheduler

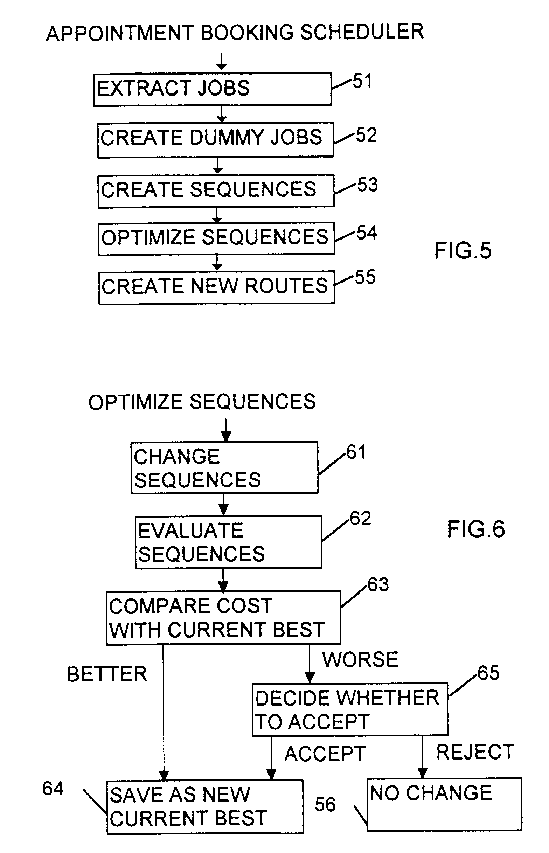

Figure 5 shows the operation of the appointment booking scheduler.

(Step 51) The scheduler first extracts the jobs to be scheduled from the job tables in the database. The scheduler picks out those jobs it is interested in for this run - i.e. those for a particular requested date and within a particular requested geographical area. Other tasks such as pre-allocated work and holiday activities are also read in by the scheduler from the database. This includes any unavailability periods that may have been input through the Gantt or database maintenance screens.

(Step 52) The scheduler then calculates the total amount of free time, by calculating the total time currently used by jobs and subtracting this from the total time available to all engineers working on the day. This free time is then divided up amongst the regions in the form of "dummy" jobs, such that each region receives the same amount of free time. This ensures that some free time is always kept in each region for the use of subsequent job offers.

(Step 53) The scheduler then takes the set of jobs to be scheduled (including the dummy jobs), and randomly allocates jobs to resources (operatives) for specific times, accounting for travel times, to create a set of sequences of jobs. The allocation of jobs to resources is performed from scratch, taking no account of the current routes stored in the database.

(Step 54) The scheduler then attempts to optimize these sequences, by minimising a cost function. One of the factors in this cost function is travel time between jobs. As a result, the optimization process will tend to group together jobs in the same region. The optimization process is described in detail below with reference to Figure 6.

(Step 55) The scheduler then uses the optimized sequences of jobs output from the optimization process to create a new table 30 representing a new set of routes. Each sequence of jobs results in a separate route in the route table. Creation of a route from a sequence of jobs involves:
- scanning the sequence of jobs to find groups of consecutive jobs in the sequence that all relate to the same region,
- calculating the "start time", "end time", "time used" and "time left" for that region, and
- creating a record for the region in the route table.

As an example, consider the following sequence of jobs:
- Job 1 :: Region A, at 9.00, duration 10 mins, travel 5 mins.
- Job 2 :: Region A, at 9.15, duration 20 mins, travel 5 mins.
- Job 3 :: Region A, Dummy job, at 9.40, duration 15 mins, travel 5 mins.
- Job 4 :: Region B, Dummy job, at 10.00, duration 25 mins, travel 5 mins.
- Job 5 :: Region B, Dummy job, at 10.30, duration 25 mins, travel 5 mins.
- Job 6 :: Region C, at 11.00, duration 15 mins, travel 5 mins.

This will result in a route as follows:
- Region A: Start 9.00 End 10.00 Time used 40 mins Time available 20 mins.
- Region B: Start 10.00 End 11.00 Time used 0 mins Time available 60 mins.
- Region C: Start 11.00 End 12.00 Time used 20 mins Time available 40 mins

### Optimization process

The appointment booking scheduler 13 uses a simulated annealing process to attempt to optimize the sequences of jobs. This employs a simulated "temperature" which is initially set a high value, and gradually reduced until it reaches a predetermined final value. At each value of the simulated temperature, the process shown in Figure 5 is performed a predetermined number of times N. The value of N can be varied according to the degree of optimization desired: a high value of N gives better optimization, but means that the scheduler takes longer to run.

At each iteration of this process, the scheduler stores the "current best" set of sequences of jobs, along with a corresponding "current best" cost value for that set of sequences. Referring to Figure 6, each iteration of the process consists of the following steps.

(Step 61) The sequences of jobs are changed in some random way, e.g. by changing the order of two adjacent jobs in a sequence, or by swapping jobs between two sequences.

(Step 62) The changed set of sequences is then evaluated to determine a cost function for the changed set of sequences. This cost function includes factors such as time taken to travel between jobs, penalties for breaking an appointment, overtime working for operatives, and achieving a suitable match between the operatives' skills and the difficulty of the jobs.

(Step 63) The evaluated cost for the changed set of sequences is then compared with the stored current best value.

(Step 64) If the evaluated cost is better (i.e. less) than the current best, the changed set of sequences is saved as the new current best set of sequences, and its cost is saved as the new current best cost.

(Step 65) If on the other hand the evaluated cost is greater than or equal to the current best value, the scheduler makes a random choice as to whether or not to accept this changed set of sequences, with a probability determined by the current simulated temperature: the higher the simulated temperature, the more likely the scheduler is to accept the changed set of sequences. If the scheduler decides to accept the changed set of sequences, the changed set of sequences is saved as the new current best set of sequences, and its cost is saved as the new current best cost.

(Step 66) If the changed set of sequences is not accepted, the existing current best is retained.

### Reactive scheduler

The reactive scheduler 14 is similar to the appointment booking scheduler, the main difference being that, whereas the appointment booking scheduler starts from a random initial allocation of jobs to resources, the reactive scheduler starts from the latest best allocation, as held in the database.

The reactive scheduler takes over from the appointment booking scheduler when a predetermined horizon is crossed (e.g. on the day that the appointments are due), and takes account of last minute variations in resource availability, appointment cancellations, and urgent jobs required to be performed as soon as possible, by rescheduling dynamically.

### Gantt manager

The Gantt manager interface 15 allows the current routes to be viewed graphically in bar chart form, to provide an overview of the operation of the schedulers.

### Watchdog

The watchdog 16 continuously monitors the status of all jobs and can initiate the appointment booking scheduler automatically if the number of appointments booked on a particular route exceeds a predetermined threshold level.

The watchdog also monitors the status of all jobs within the reactive scheduler horizon, and provides a warning of jeopardy situations.

### Some possible modifications

It will be appreciated that many modifications may be made to the system described above without departing from the scope of the present invention as defined in the appended claims. For example, instead of using a separate appointment booking scheduler and reactive scheduler, both functions may be performed by the same unit operating in different modes.

## Claims

1. A computerised appointment booking and scheduling apparatus for scheduling a plurality of operatives travelling to sites in a geographical area, the apparatus comprising a database (11) containing a pool of jobs, input means (12) for adding new jobs to said pool, and scheduling means (13) for using the pool of jobs to generate and to store in the database a plurality of routes (31), indicating sequences of sites to be visited by said operatives, characterised in that:
(a) said geographical area is divided into a plurality of regions, each of which regions includes a plurality of said sites;
(b) each of said routes (31) comprises information specifying a sequence of said regions to be visited by a particular operative, the times the operative is scheduled to enter and leave each of said sequence of regions, and the amount of free time available for new appointments in each of said sequence of regions;
(c) said input means (12) comprises means (41) for offering appointments at specific times, using the currently stored routes to check for availability of operatives in specific regions at specific times, and for updating said pool of jobs and said routes to reflect new appointments that have been accepted; and
(d) the apparatus also includes means (42,43) for periodically running said scheduling means to generate from scratch a new plurality of routes, optimising the new plurality of routes to minimise a cost function, and storing them in the database to replace the currently stored routes.

2. Apparatus according to Claim 1 wherein said means (42,43) for periodically running said scheduling means comprises means (42) for checking whether a predetermined trigger number of appointments has been made since the last time the scheduling means was run and, if so, for calling the scheduling means.

3. Apparatus according to Claim 1 or 2 wherein said scheduling means (13) comprises means (52) for generating a set of dummy jobs, collectively representing the total amount of free time available to operatives to handle new appointments, and means (53-55) for using these dummy jobs in generating said new plurality of routes to determine distribution of the free time within said new plurality of routes.

4. Apparatus according to Claim 3 wherein said scheduling means comprises means for allocating said dummy jobs between said regions to achieve a substantially even distribution of available free time between said regions.

5. A method of operating a computer apparatus to schedule a plurality of operatives travelling to sites in a geographical area, the method comprising maintaining a pool of jobs in a database (11), adding new jobs to said pool, and using the pool of jobs to generate and to store in the database a plurality of routes (31), indicating sequences of sites to be visited by said operatives, characterised in that:
(a) said geographical area is divided into a plurality of regions, each of which regions includes a plurality of said sites;
(b) each of said routes (31) comprises information specifying a sequence of said regions to be visited by a particular operative, the times the operative is scheduled to enter and leave each of said sequence of regions, and the amount of free time available for new appointments in each of said sequence of regions;
(c) the step of adding new jobs to said pool comprises offering appointments at specific times, using the currently stored routes to check for availability of operatives in specific regions at specific times, and updating said pool of jobs and said routes to reflect new appointments that have been accepted; and
(d) the method further comprises periodically running said scheduling means to generate a new plurality of routes from scratch, optimising the new plurality of routes to minimise a cost function, and storing them in the database to replace the currently stored routes.

6. A method according to Claim 5 wherein said step of periodically running said scheduling means comprises checking whether a predetermined trigger number of appointments has been made since the last time the scheduling means was run and, if so, calling the scheduling means.

7. A method according to Claim 5 or 6, further including the step of generating a set of dummy jobs, collectively representing the total amount of free time available to operatives to handle new appointments, and using these dummy jobs in generating said new plurality of routes to determine distribution of the free time within said new plurality of routes.

8. A method according to Claim 7, further including the step of allocating said dummy jobs between said regions to achieve a substantially even distribution of available free time between said regions.

## Patentansprüche

1. Rechnergesteuertes Terminbuchungs- und Ablaufplanungssystem zum Planen einer Vielzahl von Beschäftigten, die an Standorte in einem geographischen Gebiet reisen, mit einer Datenbank (11), die einen Pool von Arbeitsplätzen enthält, einer Eingabevorrichtung (12), mit der neue Arbeitsplätze dem Pool hinzugefügt werden, und einer Ablaufplanungsvorrichtung (13), um den Pool von Arbeitsplätzen zu benutzen, damit in der Datenbank eine Vielzahl von Routen (31) erstellt und gespeichert werden, die Folgen von Standorten, die von den Beschäftigten zu besuchen sind, anzeigen, **dadurch gekennzeichnet,** daß
(a) das geographische Gebiet in eine Vielzahl von Bereichen unterteilt ist, deren jedes eine Vielzahl der Standorte enthält,
(b) jede der Routen (31) Informationen enthält, die eine Folge der von einem bestimmten Beschäftigten zu besuchenden Bereiche, die Zeiten, in denen der Beschäftigte geplant hat, jeden der Folge von Bereichen zu betreten und zu verlassen, und die Menge an freier Zeit, die für neue Termine in jedem der Folge von Bereichen verfügbar ist, spezifiziert,
(c) die Eingabevorrichtung (12) eine Vorrichtung (41) aufweist, um Termine zu bestimmten Zeiten anzubieten, die laufend gespeicherten Routen zur Prüfung auf Verfügbarkeit von Beschäftigten in bestimmten Bereichen und zu bestimmten Zeiten zu benutzen, und den Pool von Arbeitsstellen und die Routen fortzuschalten, um neue Termine, die angenommen worden sind, zu berücksichtigen, und
(d) eine Einrichtung (42, 43) zum periodischen Durchlaufen der Ablaufplanungsvorrichtung vorgesehen ist, um von neuem eine neue Vielzahl von Routen herzustellen, die neue Vielzahl von Routen zur Minimierung einer Kostenfunktion zu optimieren, und sie in der Datenbank zu speichern, um die laufend gespeicherten Routen zu ersetzen.

2. System nach Anspruch 1, bei dem die Einrichtung (42, 43) zum periodischen Durchlaufen der Ablaufplanungsvorrichtung eine Vorrichtung (42) aufweist, die prüft, ob eine vorbestimtme Triggerzahl von Terminen festgelegt worden ist, seit die Ablaufplanungsvorrichtung das letzte Mal durchlaufen worden ist, und, wenn dies der Fall ist, die Ablaufplanungsvorrichtung anzurufen.

3. System nach Anspruch 1 oder 2, bei dem die Ablaufplanungsvorrichtung (13) eine Vorrichtung (52) zum Erzeugen eines Satzes von Pseudo-Arbeitsstellen, die gemeinsam die gesamte Menge an freier Zeit darstellen, die für die Beschäftigten zur Verfüging steht, um neue Termine wahrzunehmen, sowie eine Vorrichtung (53 - 55), um diese Pseudo-Arbeitsstellen zu verwenden, um die neue Vielzahl von Routen zu erstellen, damit eine Verteilung der freien Zeit innerhalb der neuen Vielzahl von Routen festgelegt wird, aufweist.

4. System nach Anspruch 3, bei dem die Ablaufplanungsvorrichtung eine Vorrichtung zum Zuordnen der Pseudo-Arbeitsstellen zwischen diesen Bereichen aufweist, um eine im wesentlichen gleichförmige Verteilung von frei verfügbarer Zeit zwischen diesen Bereichen zu erzielen.

5. Verfahren zum Betreiben eines Rechnersystems für das Planen einer Vielzahl von Beschäftigten, die an verschiedene Standorte in einem geographischen Gebiet reisen, wobei ein Pool von Arbeitsstellen in einer Datenbank (11) aufrecht erhalten wird, neue Arbeitsstellen dem Pool hinzugefügt werden, und der Pool von Arbeitsstellen verwendet wird, um in der Datenbank eine Vielzahl von Routen (31) zu erstellen und zu speichern, die Folgen von Standorten anzeigen, die von den Beschäftigten besucht werden sollen, dadurch gekennzeichnet, daß
(a) das geographische Gebiet in eine Vielzahl von Bereichen unterteilt wird, deren jeder eine Vielzahl von Standorten umfaßt,
(b) jede der Routen (31) Informationen enthält, die eine Folge der von einem bestimmten Beschäftigten zu besuchenden Bereiche, die Zeiten, in denen der Beschäftigte geplant hat, jeden der Folge von Bereichen zu betreten und zu verlassen, und die Menge an freier Zeit, die für neue Termine in jedem der Folge von Bereichen verfügbar ist, spezifiziert,
(c) das Hinzufügen neuer Arbeitsplätze zu dem Pool das Anbieten von Terminen zu bestimmten Zeiten, das Benutzen der laufend gespeicherten Routen zur Prüfung, ob der Beschäftigte in bestimmten Bereichen zu bestimmten Zeiten verfügbar ist, und das Fortschalten des Pools von Arbeitsstellen und der Routen zum Berücksichtigen neuer Termine, die angenommen worden sind, umfaßt, und
(d) die Ablaufplanungsvorrichtung periodisch durchlaufen wird, um eine neue Vielzahl von Routen von Beginn an zu erzeugen, die neue Vielzahl von Routen zu optimieren, um die Kosten zu minimieren, und sie in der Datenbank zu speichern, um die jeweils gespeicherten Routen zu ersetzen.

6. Verfahren nach Anspruch 5, bei dem das periodische Durchlaufen der Ablaufplanungsvorrichtung das Prüfen umfaßt, ob eine vorbestimmte Triggerzahl von Terminen vereinbart worden ist, seit die Planungsvorrichtung zuletzt durchlaufen worden ist, und, wenn dies der Fall ist, die Ablaufplanungsvorrichtung anzurufen.

7. Verfahren nach Anspruch 5 oder 6, bei dem ein Satz von Pseudo-Arbeitsstellen geschaffen wird, die gemeinsam die gesamte Menge an freier, für Beschäftigte zur Handhabung neuer Termine verfügbarer Zeit darstellen, und diese Pseudo-Arbeitsstellen zur Erstellung der neuen Vielzahl von Routen verwendet werden, um eine Verteilung der freien Zeit innerhalb der neuen Vielzahl von Routen zu bestimmen.

8. Verfahren nach Anspruch 7, bei dem die Pseudo-Arbeitsstellen zwischen den Bereichen zugewiesen werden, um eine im wesentlichen gleichförmige Verteilung von verfügbarer freier Zeit zwischen den Bereichen zu erzielen.

## Revendications

1. Appareil de réservation et de planification de rendez-vous par ordinateur pour planifier une pluralité d'agents se déplaçant jusqu'à des sites dans une zone géographique, l'appareil comprenant une base de données (11) contenant un groupe de tâches, un moyen d'entrée (12) pour additionner de nouvelles tâches audit groupe et un moyen de planification (13) pour utiliser le groupe de tâches afin de générer et de stocker dans la base de données une pluralité de chemins (31) indiquant des séquences de sites à visiter par lesdits agents, caractérisé en ce que :
(a) ladite zone géographique est divisée selon une pluralité de régions dont chacune inclut une pluralité desdits sites ;
(b) chacun desdits chemins (31) comprend une information qui spécifie une séquence desdites régions à visiter par un agent particulier, les instants auxquels l'agent est planifié pour entrer dans chacune de ladite séquence de régions et pour la quitter et la quantité de temps libre disponible pour de nouveaux rendez-vous dans chacune de ladite séquence de régions ;
(c) ledit moyen d'entrée (12) comprend un moyen (41) pour offrir les rendez-vous à des instants spécifiques en utilisant les chemins stockés présentement afin de vérifier la disponibilité d'agents dans des régions spécifiques à des instants spécifiques et pour mettre à jour ledit groupe de tâches et lesdits chemins pour refléter de nouveaux rendez-vous qui ont été acceptés ; et
(d) l'appareil inclut également un moyen (42, 43) pour dérouler périodiquement ledit moyen de planification afin de générer à partir de zéro une nouvelle pluralité de chemins, pour optimiser la nouvelle pluralité de chemins afin de minimiser une fonction de coût et pour les stocker dans la base de données afin de remplacer les chemins stockés présentement.

2. Appareil selon la revendication 1, dans lequel ledit moyen (42, 43) permettant de dérouler périodiquement ledit moyen de planification comprend un moyen (42) pour vérifier si oui ou non un nombre de déclenchements prédéterminé de rendez-vous a été réalisé depuis la dernière fois où le moyen de planification a été déroulé et s'il en est ainsi, pour appeler le moyen de planification.

3. Appareil selon la revendication 1 ou 2, dans lequel ledit moyen de planification (13) comprend un moyen (52) pour générer un jeu de tâches fictives représentant de façon collective la quantité totale de temps libre disponible pour que des agents manipulent de nouveaux rendez-vous et un moyen (53-55) pour utiliser ces tâches fictives lors de la génération de ladite nouvelle pluralité de chemins afin de déterminer une distribution du temps libre dans ladite nouvelle pluralité de chemins.

4. Appareil selon la revendication 3, dans lequel ledit moyen de planification comprend un moyen pour allouer lesdites tâches fictives entre lesdites régions afin de réaliser une distribution sensiblement uniforme du temps libre disponible entre lesdites régions.

5. Procédé d'activation d'un appareil d'ordinateur afin de planifier une pluralité d'agents se déplaçant jusqu'à des sites dans une zone géographique, le procédé comprenant le maintien d'un groupe de tâches dans une base de données (11), l'addition de nouvelles tâches audit groupe et l'utilisation du groupe de tâches afin de générer et de stocker dans la base de données une pluralité de chemins (31) indiquant des séquences de sites à visiter par lesdits agents, caractérisé en ce que :
(a) ladite zone géographique est divisée selon une pluralité de régions dont chacune inclut une pluralité desdits sites ;
(b) chacun desdits chemins (31) comprend une information qui spécifie une séquence desdites régions à visiter par un agent particulier, les instants auxquels l'agent est planifié pour entrer dans chacune de ladite séquence de régions et pour la quitter et la quantité de temps libre disponible pour de nouveaux rendez-vous dans chacune de ladite séquence de régions ;
(c) l'étape d'addition de nouvelles tâches audit groupe comprend l'offre de rendez-vous à des instants spécifiques, l'utilisation des chemins stockés présentement pour vérifier la disponibilité des agents dans des régions spécifiques à des instants spécifiques et la mise à jour dudit groupe de tâches et desdits chemins pour refléter de nouveaux rendez-vous qui ont été acceptés ; et
(d) le procédé comprend en outre le déroulement périodique dudit moyen de planification afin de générer une nouvelle pluralité de chemins à partir de zéro, l'optimisation de la nouvelle pluralité de chemins afin de minimiser une fonction de coût et leur stockage dans la base de données afin de remplacer les chemins stockés présentement.

6. Procédé selon la revendication 5, dans lequel ladite étape de déroulement périodique dudit moyen de planification comprend la vérification de si un nombre de déclenchements prédéterminé de rendez-vous a été réalisé depuis la dernière fois où le moyen de planification a été déroulé et s'il en est ainsi, l'appel du moyen de planification.

7. Procédé selon la revendication 5 ou 6, incluant en outre l'étape de génération d'un jeu de tâches fictives représentant collectivement la quantité totale de temps libre disponible pour que des agents manipulent de nouveaux rendez-vous et d'utilisation de ces tâches fictives lors de la génération de ladite nouvelle pluralité de chemins afin de déterminer une distribution du temps libre dans ladite nouvelle pluralité de chemins.

8. Procédé selon la revendication 7, incluant en outre l'étape d'allocation desdites tâches fictives entre lesdites régions afin de réaliser une distribution sensiblement uniforme du temps libre disponible entre lesdites régions.
